(19) Europäisches Patentamt
European Patent Office
Office européen des brevets

(11) Publication number: **0 390 718 A1**

(12) **EUROPEAN PATENT APPLICATION**

(21) Application number: **90500026.1**

(22) Date of filing: **21.03.90**

(51) Int. Cl.[5]: **C07D 401/12, A61K 31/415, A61K 31/445**

(30) Priority: **28.03.89 ES 8901078**

(43) Date of publication of application: **03.10.90 Bulletin 90/40**

(84) Designated Contracting States: **AT BE CH DE DK FR GB GR IT LI LU NL SE**

(71) Applicant: **FABRICA ESPANOLA DE PRODUCTOS QUIMICOS Y FARMACEUTICOS, S.A. (FAES)**
**c/ Maximo Aguirre, 14**
**E-48940 Lejona-Lamiaco (Vizcaya)(ES)**

(72) Inventor: **Orjales Venero, Aurelio**
**P. del Puerto, 24**
**Neguri (Vizcaya)(ES)**
Inventor: **Rubio Royo, Victor**
**C/Bizkerre, 30-1o.**
**Guecho (Vizcaya)(ES)**

(74) Representative: **Isern-Cuyas, Maria Luisa**
**Paseo de la Castellana 131, Bajo C**
**E-28046 Madrid(ES)**

(54) **Process for preparing new 1-diphenylmethylpiperidine derivatives.**

(57) The process to prepare new 1-diphenylmethylpiperidine derivatives, of formula (I)

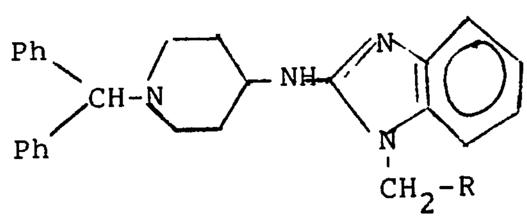

(I)

comprises reacting an N-non substituted piperidine of formula II with a diphenylmethane halogenide XCH-$(Ph)_2$ in the presence of a base and eventually a catalyst, optionally converting the free base obtained into an addition salt, with a pharmaceutically acceptable acid, through conventional treatment with the suitable acid.

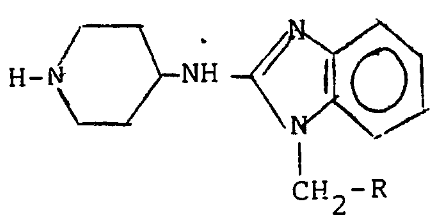

(II)

EP 0 390 718 A1

## PROCESS FOR PREPARING NEW 1-DIPHENYLMETHYLIPIPERIDINE DERIVATIVES.

The present invention describes a series of new 1-diphenylmethylpiperidine derivatives represented by formula I, their addition salts with pharmaceutically acceptable acids, the methods whereby they can be prepared and an antihistaminic pharmaceutical composition containing one such derivative as active ingredient.

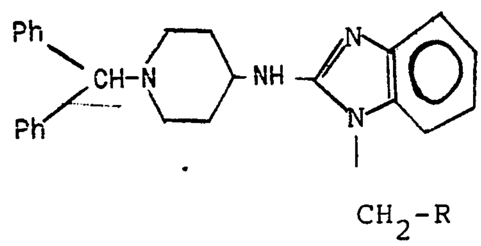

I

In formula I, R can be hydrogen, a short chain alkyl group substituted by a short chain alcoxyl group or a phenyl radical substituted in its position 4 by a halogen or a methoxy group. A short chain alkyl group is a carbonaceous radical with one to three units of both linear and branched carbon; a short chain alcoxyl group is an $OR^1$ remainder where $R^1$ has the same meaning previously allocated to a short chain alkyl group; a halogen is F, Cl, Br or I.

Allergic type processes arise when given specialized cells of the human body react in the presence of foreign substances, antigens, releasing a number of chemical agents of very different nature and importance, among them histamine, arachidonic acid metabolites, leucotriennals, prostaglandins, and so forth. The signs of the allergic response -bronchoconstriction, smarts, reddenings, inflammation and so on- result from the local effects of such agents.

As histamine is one of the most important and best known chemical agents in the allergic process, drugs capable of opposing their action, antihistaminic drugs, have been widely developed in recent years. Although it is true that really effective drugs have been found as antihistamines, the manifold and relatively important side-effects, especially as regards the central nervous system, derived from their use, make the search for new antihistaminic compounds to still be a current goal.

Classic antihistaminic compounds are characterized in that their structure has an aminoethylic radical bonded by the carbon to a large lipophile group (fig. 1), preferably the diphenylmethyl group (Devlin and Hargrave "Pulmonary and antiallergic drugs", John Wiley, 1985, 201). The aminoethylic group can have a linear nature

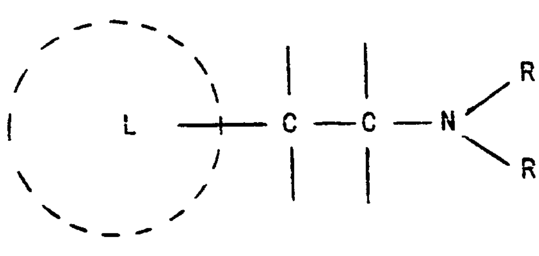

Fig. 1

as in chlorphenyramine, form part of a cyclic piperidine (for instance, terphenadine) or piperazine (for instance, cinarizine) structure or be bonded to the lipophile group by an oxygen atom (for instance, diphenhydramine). The compounds claimed herein have the novelty of presenting the diphenylmethyl group directly bonded to the piperidine nitrogen, being clearly distinguished from the classic antihistamines, and having a notable antihistaminic activity with no significant effects for the central nervous system.

The subject hereof are the aforesaid new 1-diphenylmethylpiperidine derivatives, and their addition salts with pharmaceutically acceptable acids, useful as low toxicity antihistamines, obtained by means of the

preparation processes described from the new 1-diphenylmethylpiperidine derivatives and designed to be used in pharmaceutical compositions.

The compounds described herein are the products of formula I, and their addition salts with pharmaceutically acceptable salts such as chlorhydric, bromhydric, sulphuric, nitric, phosphoric, acetic, propanoic, butanodioic, (E)-2-butenodioic, (z)-2-butenodioic, 2-oxopropanoic and like acids.

The compounds I can be suitably prepared by means of an alkylation reaction of piperidine II, where R can be hydrogen, a short chain alkyl group, a short chain alkyl group

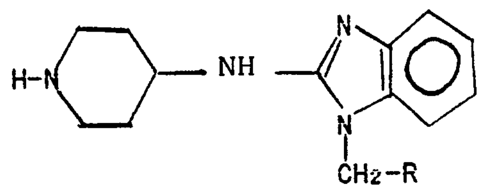

II

substituted by a short chain alcoxyl group or a phenyl radical substituted in its position 4 by a halogen or a methoxy group with a diphenylmethane halogenide III, where X is fluorine,

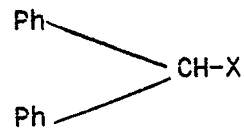

III

chlorine, bromine or iodine. The alkylation reaction is verified in the presence of an equimolecular amount or a base excess (up to 50%), that could be organic, for instance, triethylamine or pyridine or inorganic, for instance, an alkaline metal carbonate or hydrogencarbonate; addition of an alkaline metal iodide salt can improve the performance obtained. The alkylating agent III is used in an equimolecular amount regarding piperidine II or in an excess of up to 100%. Suitable solvents to carry out this type of reactions include alcohols such as methanol, ethanol, isopropanol, buthanol and the like; aromatic hydrocarbides such as benzene, methylbenzene, dimethylbenzene and the like; an ether such as tetrahydrofurane, dioxane; acetonitrile, dimethylformamide, nitrobenzene and the like, or else a mixture of some such solvents. The reaction is verified at temperatures ranging between 20°C and the boiling temperature of the solvent used.

The piperidines II used in the alkylation reaction are known products or can be prepared by means of conventional methods (for instance, lemura, J. Hetertocycl. Chem. 24, 31, 1987).

The compounds of formula I can be changed into pharmaceutically acceptable salts by treating them with a suitable acid, such as chlorhydric, bromhydric, sulphuric, phosphoric, oxalic, propanoic, butanodioic, (Z)-2-butenodioic, (E)-2-butenodioic and like acids.

However, treating one of such salts with an inorganic base provides the compounds of formula I in the form of free base.

The antihistaminic activity of the compounds hereof has been determined "in vitro" using the test model of guinea pig isolated ileum contractions caused by histamine; segments of terminal ileum were placed in an organ bath with Tyrode solution at 37°C aerated with carbogen; contractions were logged in isotonic conditions using a lever transducer; accumulative dose-response curves for the histamine were logged to find out the concentration generating a maximal response of 80%-90% in each preparation, and use the same to establish an initial valuation of the products' antihistaminic activity; after obtaining the same response twice in the presence of vehicle, increasing concentrations of the products ($10^{-8}$-$10^{-5}$ M) were added measuring the contractile response produced by histamine after 10 minutes of incubation with each drug concentration; the drug concentration inhibiting 50% of the response to the histamine ($CI_{50}$) has been calculated in each preparation. The results obtained are summed up in Table I.

TABLE I

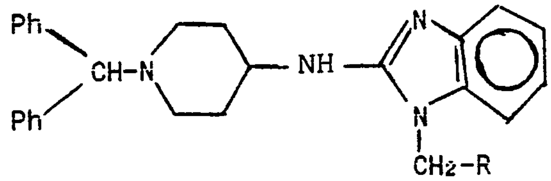

| Exp. | R | $CI_{50}$ |
|---|---|---|
| 1 | H | $2.7 \times 10^{-6}$ |
| 2 | $-CH_2CH_3$ | $5.5 \times 10^{-7}$ |
| 3 | $-CH_2OCH_2CH_3$ | $1.8 \times 10^{-7}$ |
| 4 | $4-C_6H_4-F$ | $6.3 \times 10^{-8}$ |
| 5 | $4-C_6H_4-Cl$ | $5.6 \times 10^{-8}$ |
| 6 | chlorphenyramine | $1.2 \times 10^{-5}$ |

A preliminary study has been made of the main pharmacologic effects using Swiss male mice distributed in groups of four animals; each group was administered one of the products in doses of 300 mg/kg and the animals began to be observed, logging the time when symptoms appeared or given reflexes decreased: spontaneous activity, appearance of stereotype, trembling, convulsions, abnormal body position, loss of reflexes, extent of palpebral aperture, salivation, pyloerection, hypothermia, lachrymation or change in skin colour; observation lasted for 30 minutes and was repeated 1, 2, 3, 4 and 24 hours after treatment; the reference compound chosen was a well-known antiallergic, ketotyphene. The results observed are summed up in table 2.

TABLE 2

| | | Maximum effect observed/No. of animals | | | | | | |
|---|---|---|---|---|---|---|---|---|
| Exp. | R | Death | Decreased spontaneous activity | Palpebral potosis | Hypothermia | Convulsions | Body reflex loss | Hearing reflex loss |
| 1 | $CH_2CH_3$ | - | 0 | 1/4 | 0 | - | - | - |
| 2 | $4-C_6H_4-F$ | - | 0 | 1/4 | 0 | - | - | - |
| 3 | $4-C_6H_4Cl$ | - | 0 | 0 | 0 | - | - | - |
| Ketotiphene | | - | 2/4 | 1/4 | 4/4 | - | 1/4 | - |

The results set forth in tables 1 and 2 show that the products described herein are powerful antihistamines. greater than classic antihistamines such as chlorphenyramine, and have no noticeable central effects, hence rendering the same potentially useful to be employed, under acceptable therapeutical forms, in the prevention and symptomatic treatment of allergic type diseases characterized by the release of histamine.

The compounds described herein can be expressed in different pharmaceutical forms to be subsequently administered orally or topically.

Preparations to be administered orally are made by closely mixing an effective amount of one of the products described herein with a conventional inert support such as lactose, cellulose, starch and the like or mixtures thereof, to subsequently compress or encapsulate the same. When presented in syrup, suspension or solution form, water, simple syrup, glycols, alcohols or the like are used as support, with the product

dissolved or suspended therein. Topical administration can take the form of creams, ointments and gels, using conventional agents such as vaseline, polyethylenglycol, propylenglycol and the like as support.

The following examples illustrate this invention at no event limiting its scope.

## Example 1

### Preparation of N-[1-(diphenylmethyl)piperidine-4-yl]-1-methyl-2-amino-1 H-benzimidazol.

2.3 grammes of N-(4-piperidinyl)-1-methyl-2-amino-1 H-benzimidazol are dissolved in 20 millilitres of dimethyl formamide and 2.6 grammes of bromodiphenylmethane, 1.06 grammes of sodium carbonate and 0.1 grammes of potassium iodide are then added; the resultant suspension is heated at 70° C, with vigorous stirring, for 16 hours; 20 millilitres of water are added and evaporation takes place until dry with the help of vacuum. The educt obtained comprises methylene chloride and water; the organic phase is washed with water, dried on anhydrous sodium sulphate and concentrated producing an oil that is dissolved in 10 millilitres of hot ethanol; 2.3 grammes of fumaric acid dissolved in 10 millilitres of hot ethanol are added and left to cool down with the crystallization of 1.5 grammes of N-[1 (diphenylmethyl)piperidine-4-yl]-1-methyl-2-amino-1 H-benzimidazol fumarate. MP: 124° C (decomposes).

$^1H$ RMN (DMSO-$d_6$)δ:

1.65 (m, 2$H_{piperidine}$), 1.95 (m, 4$H_{piperidine}$), 2.82 (m, 2$H_{piperidine}$), 3.5 (s, N-$CH_3$), 3.75 (m, CH-N), 4.35 (s, CH -$(Ph)_2$), 6.62 (s, 2 CH=), 6.9- 7.5 (m, 14H Ar).

$^{13}C$-RMN (DMSO-$d_6$) ppm:

28.3, 32.0, 50.1, 75.0, 107.4, 114.6, 118.6, 120.4, 126.8, 127.5, 128.6, 134.2, 134.8, 143.3, 154.0.

| Elementary analysis for $C_{26}H_{28}N_4$,$C_4H_4O_4$ | | | |
|---|---|---|---|
| calculated: | C, 70.43; | H, 6.26; | N, 10.95 |
| found: | C, 71.02; | H, 6.18; | N, 11.3 |

## Example 2

### Preparation of N-[1-(diphenylmethyl)piperidine-4-yl]-1-propyl-2-amino-1 H-benzimidazol.

2.3 grammes of N-(4-piperidinyl)-1-propyl-2-amino-1 H-benzimidazol are dissolved in 50 millilitres of acetonitrile and 8 grammes of chlorodiphenylmethane and 1.8 grammes of sodium bicarbonate added and the mixture is heated at reflow for 24 hours and is then concentrated. The educt is poured on a 10% chlorhydric acid solution and washed with methylene chloride; it is alkalinized with sodium hydroxide solution and extracted with methylene chloride. The organic solution is washed with water, dried on sodium sulphate and concentrated to obtain an oil that is converted into a fumarate as detailed in example 1, 7 grammes of N-[1 (diphenylmethyl)piperidine-4-yl]-1-propyl-2-amino-1 H-benzimidazol fumarate being obtained. MP: 196° C (decomposes).

| Elementary analysis for $C_{28}H_{32}N_4$.2 $C_4H_4O_4$: | | | |
|---|---|---|---|
| Calculated: | C, 65.94; | H, 6.10; | N, 8.55 |
| Found: | C, 66.28; | H, 5.83; | N, 8.73. |

## Example 3

Preparation of N-[1-(diphenylmethyl)piperidine-4-yl]-1-(ethoxy-ethyl)-2-amino-1 H-benzimidazol.

Method as in example no. 1, but starting from 5.76 grammes of N-(4-piperidinyl)-1-(ethoxyethyl)-2-amino-1 H-benzimidazol, 5 grammes of methane bromodiphenyl, 2.5 grammes of sodium carbonate and 0.2 grammes of potassium iodide, yielding 7.2 grammes of N-[1-(diphenylmethyl)piperidine-4-yl]-1-(ethoxy-ethyl)-2-amino-1 H-benzimidazol fumarate. MP: 200 °C (decomposes).

| Elementary analysis for $C_{29}H_{34}N_4O.C_4H_4O_4$: | | | |
|---|---|---|---|
| Calculated: | C, 69.47; | H, 6.66; | N, 9.82 |
| Found: | C, 70.35; | H, 6.81; | N, 9.70. |

Example 4

Preparation of 1-[(4-chlorophenyl)methyl]-N-[1-(diphenylmethyl)piperidine-4-yl]-2-amino-1 H-benzimidazol.

Method as in no. 2, but starting with 6.8 grammes of 1-[(4-chlorophenyl)methyl]-N-(4-piperidinyl)-2-amino-1 H-benzimidazol, yielding 7.5 grammes of 1-[(4-chlorophenyl)methyl]-N-[1-(diphenylmethyl)-piperidine-4-yl]-2-amino-1 H-benzimidazol fumarate. MP: 199 °C (decomposes).

$^1$H-RMN (DMSO-$d_6$):

1.65 (m, $2H_{piperidine}$), 1.95 (m, $4H_{piperidine}$), 2.8 (m, $2H_{piperidine}$), 3.8 (m, CH-N), 4.35 (s, CH $(PH)_2$), 5.35 (s, 2H, $CH_2$-PH-Cl), 6.65 (s, 2 CH=), 6.85-7.5 (m, 18 H Ar).

$^{13}$C-RMN (DMSO-$d_6$) ppm:

31.9, 44.0, 50.2, 50.9, 75.1, 108.3, 114.8, 119.2, 121.1, 127.0, 127.7, 128.4, 128.7, 128.9, 132.2, 133.9, 134.4, 136.1, 141.3, 143.2, 153.5, 166.5.

| Elementary analysis for $C_{32}H_{31}ClN_4$. $1.5C_4H_4O_4$ | | | |
|---|---|---|---|
| calculated: | C, 67.02; | H, 5.44; | N, 8.23 |
| found: | C, 66.80; | H, 5.31; | N, 8.19. |

Example 5

Preparation of N-[(1-diphenylmethyl)piperidine-4-yl]-1-[(4-fluorophenyl)methyl]-2-amino-1 H-benzimidazol.

Method as in example no. 2, but starting from 6.5 grammes of 1-[(4-fluorophenyl)methyl]-N-(4-piperidinyl)-2-amino-1 H-benzimidazol, yielding 6.8 grammes of N-[(1-diphenylmethyl)piperidine-4-yl]-1-[(4-fluorophenyl)methyl]-2-amino-1 H-benzimidazol fumarate. MP: 220 °C (decomposes).

$^1$H RMN (DMSO-$d_6$)δ:

1.55 (m, $2H_{piperidine}$), 1.85 (m, $4H_{piperidine}$), 2.68 (m, $2H_{piperidine}$), 3.7 (m, CH-N), 4.25 (s, 1 H, CH $(PH)_2$), 5.2 (s, 2H, $CH_2$)-Ph-F), 6.55 (s, 2 $H_{fumaric}$), 6.55-7.4 (m, 18H Ar).

$^{13}$C-RMN (DMSO-$d_6$) ppm:

31.9, 43.7, 50.0, 75.0, 108.0, 114.9, 115.3, 115.6, 118.7, 120.7, 126.8, 127.5, 128.2, 128.6, 129.0, 133.3, 134.1, 134.2, 142.1, 143.3, 153.7, 166.3.

| Elementary analysis for $C_{32}H_{31}FN_4 . C_4H_4O_4$: | | | |
|---|---|---|---|
| calculated:<br>found: | C, 71.3;<br>C, 70.85; | H, 5.77;<br>H, 5.92; | N, 9.24<br>N, 9.48. |

## Claims

1.- Process for preparing new 1-diphenylmethylpiperidine derivatives of formula

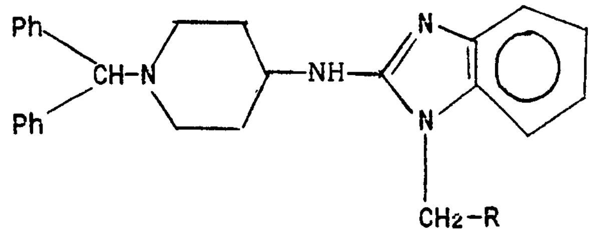

where R can be H, a short chain alkyl group, a short chain alkyl group substituted by a short chain alcoxyl group or a para-substituted phenyl radical, and their addition salts with pharmaceutically acceptable acids, characterized because it is reacted with a non-substituted N-piperidine of formula

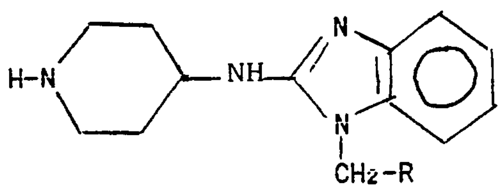

where R has the above mentioned meanings with a diphenylmethane halogenide $XCH(Ph_2)$ where X is fluorine, chlorine, bromine, or iodine, in the presence of a base and eventually a catalyst, optionally changing the free base obtained into an addition salt with a pharmaceutically acceptable acid by conventional treatment with the suitable acid.

2.- Process in accordance with claim 1 characterized in that the base used can be organic such as triethylamine or pyridine, or inorganic such as alkaline metal carbonates or bicarbonates.

3.- Process in accordance with preceding claims, characterized in using an alkaline metal iodide as catalyst.

4.- Process in accordance with preceding claims, characterized in that the new 1-diphenylmethylpiperidine derivatives are:

N-[1-(diphenylmethyl)piperidine-4-yl]-1-methyl-2-amino-1 H- benzimidazol;
N-[1-(diphenylmethyl)piperidine-4-yl]-1-propyl-2-amino-1 H-benzimidazol;
N-[1-(diphenylmethyl)piperidine-4-yl]-1-(ethoxyethyl)-2-amino-1 H-benzimidazol;
1-[(4-chlorophenyl)methyl]-N-[1-(diphenylmethyl)piperidine-4-yl]-1-2-amino-1 H-benzimidazol;
N-[1-(diphenylmethyl)piperidine-4-yl]-1-[(4-fluorophenyl)methyl]-2-amino-1 H-benzimidazol.

European Patent Office

# EUROPEAN SEARCH REPORT

Application Number

EP 90 50 0026

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 282 133 (JANSSEN PHARMACEUTICA N.V.)<br>--- | | C 07 D 401/12<br>A 61 K 31/415<br>A 61 K 31/445 |
| D,A | JOURNAL OF HETEROCYCLIC CHEMISTRY, vol. 24, no. 1, January/February 1987, pages 32-37, Provo, US; R. IEMURA et al.: "Synthesis of benzimidazole derivatives as potential H1-antihistaminic agents"<br>* The whole document *<br>----- | | |

| TECHNICAL FIELDS SEARCHED (Int. Cl.5) |
|---|
| C 07 D 401/00<br>A 61 K 31/00 |

The present search report has been drawn up for all claims

| Place of search | Date of completion of the search | Examiner |
|---|---|---|
| THE HAGUE | 21-06-1990 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)